# EUROPEAN PATENT SPECIFICATION

(11) **EP 2 050 480 B1**
(45) Date of publication and mention of the grant of the patent: **05.03.2014**
(21) Application number: 07791848.0
(22) Date of filing: 02.08.2007
(51) Int. Cl.: A61N 1/04, A61N 1/32, H01R 25/00

(54) **CONDUCTION CORD FOR LOW FREQUENCY TREATMENT DEVICE AND ELECTRODE PAD HAVING THE CONDUCTION CORD ATTACHED THERETO**
LEITUNGSKABEL FÜR NIEDERFREQUENZBEHANDLUNGSGERÄT UND ELEKTRODE MIT DARAN BEFESTIGTEM LEITUNGSKABEL
CÂBLE CONDUCTEUR POUR DISPOSITIF DE TRAITEMENT BASSE FRÉQUENCE ET PATIN DE CONNEXION D'ÉLECTRODE PRÉSENTANT LE CÂBLE CONDUCTEUR FIXÉ À CELUI-CI

(30) Priority: 08.08.2006 JP 2006215763
(43) Date of publication of application: 22.04.2009
(73) Proprietor: Omron Healthcare Co., Ltd., Kyoto 615-0084 (JP)
(72) Inventor: MIKI, Akitoshi, Kyoto-shi, Kyoto 6150084 (JP); ASAI, Rika, Kyoto-shi, Kyoto 6150084 (JP); MIZUTA, Yoshikazu, Kyoto-shi, Kyoto 6150084 (JP)
(74) Representative: Kilian Kilian & Partner
(86) International application number: PCT/JP2007/065168
(87) International publication number: WO 2008/018359

(56) References cited:
- DE-A1- 3 813 838
- GB-A- 2 160 427
- JP-A- 07 059 866
- JP-A- 2000 126 312
- JP-A- 2000 126 312
- JP-A- 2003 093 522
- JP-A- 2005 006 982
- JP-U- 61 018 151

## Description

### TECHNICAL FIELD

The present invention relates to a conduction cord for low frequency treatment device and an electrode pad attached with the same.

### BACKGROUND ART

The following are conventionally known for a conduction cord for low frequency treatment device and an electrode pad attached with the same.

In other words, a conduction cord is known in which, in order to be easily attached to a site having a steep curved surface through an adhesive pad, a pair of connection terminals for connecting a conductive section of one adhesive pad to a two-fraction adhesive pad is arranged on one surface, a groove is arranged between the pair of connection terminals arranged on one surface, the pair of connection terminals are arranged such that the respective axial lines are inclined, and at least the periphery of the groove is formed using an easily bendable material (refer to Patent Document 1).

Patent Document 1: Japanese Unexamined Patent Publication No. 2002-191704

### DISCLOSURE OF THE INVENTION

However, in such conventional technique, the response capability to the curved surface to attach the adhesive pad to the site having a steep curved surface is limited even if an easily bendable material is used, the groove is arranged between the pair of connection terminals, or the pair of connection terminals are arranged such that the respective axis lines are inclined. Furthermore, with the adhesive pad in which the conductive section is divided into three or more portions, the connecting portion of the conduction cord and the adhesive pad becomes large, and attachment to the site having a steep curved surface becomes more difficult.

If such an adhesive pad is attached to a treatment site having a large curvature such as wrist, the ends of the adhesive pad lifts up, and intimate attachment of the adhesive pad to the treatment site of large curvature becomes more difficult.

The present invention has been made considering the above circumstance, it is an object of the present invention to enable a pad to be more reliably and intimately attached to the treatment site even when attaching the pad to the treatment site of large curvature.

The present invention adopts the following configuration to achieve the above object.

Specifically, a conduction cord for low frequency treatment device according to the present invention includes a plurality of connection terminal portions arranged to contact to and separate from a plurality of electrodes arranged on a pad; a plurality of lead wires, connected to each of the plurality of connection terminal portions, for electrically connecting a low frequency treatment device body and the pad; and a coupling portion, made from an elastic material, for coupling the connection terminal portions; wherein the plurality of connection terminal portions are independently arranged, and coupled by the coupling portion to form one connection terminal portion group.

The flexibility between the connection terminal portions can be enhanced by coupling and connecting the plurality of connection terminal portions, which are independently arranged, by the coupling portion and forming one connection terminal portion group. Thus, even with the pad connected (attached) with the conduction cord for low frequency treatment device according to the present invention, the flexibility can be maintained without the movement regulated by the conduction cord as in the prior art.

Therefore, even if the pad connected with the conduction cord for low frequency treatment device according to the present invention is attached to a treatment site of large curvature such as a wrist, the ends of the pad does not lift up, and the pad can be more reliably and intimately attached to the treatment site.

The coupling portion preferably has a curved shape between the connection terminal portions.

With the coupling portion formed to a curved shape, greater stretchability and flexibility can be provided to the coupling portion. Therefore, even if the pad connected with the conduction cord for low frequency treatment device according to the present invention is attached to a treatment site of large curvature such as a wrist, the coupling portion can flexibly deform according to the movement of the pad without regulating the movement of the pad.

The coupling portion preferably has a curved shape between the connection terminal portions in a natural state not applied (acted) with external force, but the coupling portion between the connection terminal portions may have a curved shape in a state the plurality of connection terminal portions are connected to the pad before being attached to the treatment site of large curvature (before being bent). That is, the coupling portion has a curved shape as a shape that does not inhibit the bending movement of the pad, and that further reduces the external force applied on the pad even in a state the pad is attached (bent) to the treatment site of large curvature.

The coupling portion may be arranged in a curved state to enable a user to insert a finger between the coupling portion and the pad and hold the pad with the plurality of connection terminal portions connected to the plurality of electrodes arranged on the pad.

According to such a configuration, the attachment workability in attaching the pad connected with the conduction cord for low frequency treatment device according to the present invention to the treatment site can be enhanced. Furthermore, the pad can be more reliably attached to the desired treatment site since the finger (fingertip) of the user is positioned at the coupling portion arranged between the electrodes.

The coupling portion may be arranged in a curved state to enable a user to hold the pad between the fingers with the plurality of connection terminal portions connected to the plurality of electrodes arranged on the pad.

According to such a configuration, the attachment workability in attaching the pad connected with the conduction cord for low frequency treatment device according to the present invention to the treatment site can be enhanced. Since the user can hold the coupling portion arranged between the electrodes between the fingers, the convenience in handling the pad can be further enhanced and the pad can be more reliably attached to the desired treatment site.

A uniting means for uniting the plurality of lead wires from the low frequency treatment device body side to the vicinity of the connection terminal portion group is further arranged; wherein the plurality of lead wires may be branched one by one at the vicinity of the connection terminal portion group, and connected to each of the plurality of connection terminal portions.

According to such a configuration, the plurality of lead wires can be united to one, and thus the plurality of lead wires does not tangle, and the low frequency treatment device can be handled more satisfactorily. The lead wire does not regulate the movement of the connection terminal portion since the lead wires are branched one by one at the vicinity of the connection terminal portion group, and respectively connected to the plurality of connection terminal portions. That is, even if the pad connected with the conduction cord for low frequency treatment device according to the present invention is attached to the treatment site of large curvature such as a wrist, the lead wire does not regulate the movement of the pad.

A uniting means for uniting the plurality of lead wires from the low frequency treatment device body side to the connection terminal portion group is further arranged; wherein the plurality of lead wires united to the connection terminal portion group by the uniting means may be arranged so as to pass through an interior of the coupling portion and connect to the corresponding connection terminal portion.

According to such a configuration, the plurality of lead wires are united to one, and thus the plurality of lead wires do not tangle, and the low frequency treatment device can be handled more satisfactorily. The plurality of lead wires united to the connection terminal portion group by the uniting means pass through the interior of the coupling portion and connect to the corresponding connection terminal portion, and thus the plurality of lead wires does not tangle around the finger, and the convenience and the handleability in handling the low frequency treatment device can be further enhanced.

When the lead wire is passed through the interior of the coupling portion, this is not limited to a state in which the outer periphery of the lead wire is completely covered (wrapped) by the coupling portion, and may be a state in which at least one part of the outer periphery of the lead wire is covered by the coupling portion.

A projection projecting to be held by the finger may be arranged on a side opposite to the side connecting to the plurality of electrodes arranged on the pad at the plurality of connection terminal portions.

According to such a configuration, the user can grip the projection when handling the conduction cord, and thus the convenience and the handleability in handling the conduction cord can be further enhanced. Furthermore, since the projection (gripping portion) is arranged at a connecting portion between the conduction cord and the pad, the attachment workability in attaching the conduction cord to the pad can be further enhanced. Moreover, the attachment work in attaching the pad connected with the conduction cord for low frequency treatment device according to the present invention to the treatment site is more easily carried out.

The plurality of connection terminal portions may configure one connection terminal portion group formed in a line.

A direction in which the plurality of lead wires united by the uniting means is directed towards the connection terminal portion group may be orthogonal to an aligned direction of the connection terminal portion group formed in a line.

According to such a configuration, the attachment work inn attaching the pad connected with the conduction cord for low frequency treatment device according to the present invention to the treatment site is more easily carried out.

At least one connection terminal portion of the plurality of connection terminal portions may be arranged to be movable with respect to the coupling portion.

According to such a configuration, even if the pad connected with the conduction cord for low frequency treatment device according to the present invention is attached to the treatment site of large curvature such as a wrist, the pad can be more reliably and intimately attached to the treatment site since the connection terminal portion can be moved with respect to the coupling portion in addition to the flexibility of the coupling portion.

An electrode pad according to the present invention is a pad attached with the conduction cord for low frequency treatment device described above, the pad including a plurality of conductive electrode layers, arranged on a base material sheet, respectively configuring an electrode connected to the plurality of connection terminal portions; and a gel layer having an adhesive surface integrally formed by gel-like material, arranged so as to cover the plurality of conductive electrode layers on the base material sheet, and provided for attachment to a treatment site.

According to such a configuration, greater adhesive region with respect to the treatment site can be ensured, and thus the adhesion of the pad with respect to the treatment site can be increased. Therefore, even if the electrode pad according to the present invention is attached to the treatment site of large curvature such as a wrist, the ends of the pad does not lift up, and the pad can be more reliably and intimately attached to the treatment site.

The gel layer does not need to be arranged for every conductive electrode layer, and the generation of the gel layer can be simplified by integrally arranging the gel layer on the base material sheet. Furthermore, the shape of the conductive electrode layer may be complexified since the gel layer does not need to be arranged for every conductive electrode layer (separation and generation). That is, the shape of the conductive electrode layer can be appropriately set according to the specification of the low frequency treatment device.

The rigidity of the electrode pad body becomes substantially even since the adhesive portion with respect to the treatment site spreads over the entire base material sheet by integrally arranging the gel layer on the base material sheet. Thus, even if the electrode pad body is bent, the portion that easily folds is eliminated. Therefore, when attaching the pad to the treatment site, the possibility of the pad being folded and the adhesive surfaces sticking to each other is further lowered.

Each configuration above can be combined as much as possible for use.

### EFFECT OF THE INVENTION

As described above, according to the present invention, the pad can be more reliably and intimately attached to the treatment site even when attaching the pad to the treatment site of large curvature.

### BRIEF DESCRIPTION OF THE DRAWINGS

Fig. 1 is a perspective view schematically showing a pad and a cord in a first embodiment of the present invention.
Fig. 2 is a plan view schematically showing the pad and the cord in the first embodiment of the present invention.
Fig. 3 is a view schematically showing a cross section taken along line A-A in Fig. 2.
Fig. 4 is an enlarged view of portion B shown in Fig. 3.
Fig. 5 is a perspective view schematically showing the pad in the first embodiment of the present invention.
Fig. 6 schematically shows a view, seen from an adhesive surface side, of the pad in the first embodiment of the present invention.
Fig. 7 is a view describing a variant on the arrangement of the conductive carbon layers.
Fig. 8 is a view describing a variant on the arrangement of the conductive carbon layers.
Fig. 9 is a view describing a variant on the arrangement of the conductive carbon layers.
Fig. 10 is a perspective view schematically showing an outer appearance of a low frequency treatment device in the first embodiment of the present invention.
Fig. 11 is a perspective view schematically showing a cord in a second embodiment of the present invention.
Fig. 12 is a side view schematically showing the pad and the cord in the second embodiment of the present invention.
Fig. 13 is a perspective view schematically showing a cord in a third embodiment of the present invention.
Fig. 14 is a side view schematically showing the pad and the cord in the third embodiment of the present invention.
Fig. 15 is a perspective view schematically showing a cord in a fourth embodiment of the present invention.
Fig. 16 is a perspective view schematically showing a cord in a fifth embodiment of the present invention.
Fig. 17 is a side view schematically showing the pad and the cord in the fifth embodiment of the present invention.
Fig. 18 is a view schematically showing a variant in the fifth embodiment of the present invention.
Fig. 19 is a perspective view schematically showing a cord in a sixth embodiment of the present invention.
Fig. 20 is a side view schematically showing the pad and the cord in the sixth embodiment of the present invention.
Fig. 21 is a plan view schematically showing a cord in a seventh embodiment of the present invention.
Fig. 22 is a side view schematically showing the pad and the cord in the seventh embodiment of the present invention.
Fig. 23 schematically shows a view seen from the connecting portion side with the pad of the cord in the seventh embodiment of the present invention.
Fig. 24 is a view schematically showing a state in which the user holds the pad by hand in the seventh embodiment of the present invention.
Fig. 25 is a view schematically showing a state in which the user holds the pad by hand in the seventh embodiment of the present invention.
Fig. 26 is a plan view schematically showing a cord in an eighth embodiment of the present invention.
Fig. 27 is a side view schematically showing the pad and the cord in the eighth embodiment of the present invention.

### DESCRIPTION OF SYMBOLS

- 10: Low frequency treatment device
- 20: Treatment device body
- 21: Button
- 22: Display unit
- 30: Pad
- 31: Adhesive surface
- 32: Gel
- 33a, 33b, 33c: Conductive carbon layer
- 34: Base material sheet
- 40, 50, 60, 70, 80, 90, 100, 110: Cord
- 41: Plug
- 42 (42a, 42b, 42c): Connection terminal portion
- 43a, 43b, 43c: Lead wire
- 44, 51, 61, 71, 81, 91, 101, 111: Coupling portion
- 45: Branching portion
- 46a, 46b, 46c: Snap
- 52, 92: Groove
- 72: Projection
- 102: Gap

### BEST MODE FOR CARRYING OUT THE INVENTION

Best modes for carrying out the invention will be specifically described in an illustrative manner based on embodiments with reference to the drawings. The dimension, material, shape, relative arrangement, and the like of the components described in the embodiment are not intended to limit the scope of the invention unless otherwise specifically stated.

### (First Embodiment)

A conduction cord for low frequency treatment device and an electrode pad according to a first embodiment of the present invention will be described below.

First, a low frequency treatment device applied with the conduction cord for low frequency treatment device and the electrode pad according to the first embodiment of the present invention will be discussed.

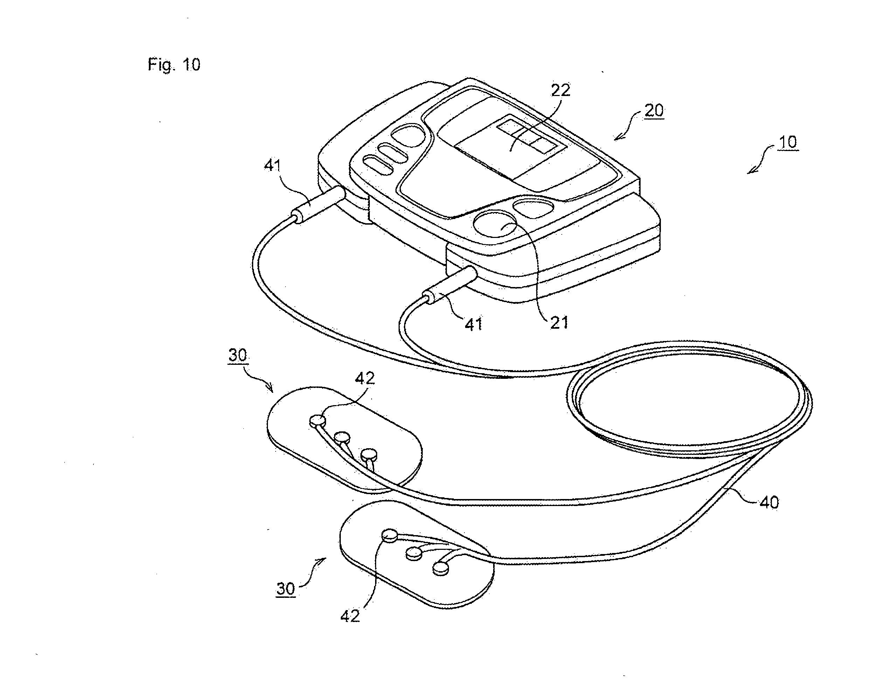

Fig. 10 is a perspective view schematically showing an outer appearance of a low frequency treatment device 10 in the first embodiment of the present invention.

The low frequency treatment device 10 is schematically configured by a treatment device body 20, a pair of pads 30 to be attached to a treatment site, and a cord 40 for electrically connecting the treatment device body 20 and the pad 30. The pad 30 serves as the electrode pad of the present invention, and the cord 40 serves as the conduction cord for low frequency treatment device of the present invention.

The treatment device body 20 has a plurality of buttons 21 provided for power supply and various settings, and a display unit 22 for displaying set content and treatment time. Various techniques including the known technique may be applied to the treatment device body itself, and thus a detailed description thereof will not be given.

The pad 30 serves as an electrode pad of the first embodiment of the present invention, and is made by a thin and highly flexible member, where one surface is made from a gel-like conductive material and has adhesiveness.

The cord 40 serves as the conduction cord for low frequency treatment device of the first embodiment of the present invention, where a plug 41 for connecting to the treatment device body 20 is arranged at one end, and a connection terminal portion 42 for connecting to the pad 30 is arranged at the other end.

The pad 30 and the cord 40 will be further described in detail.

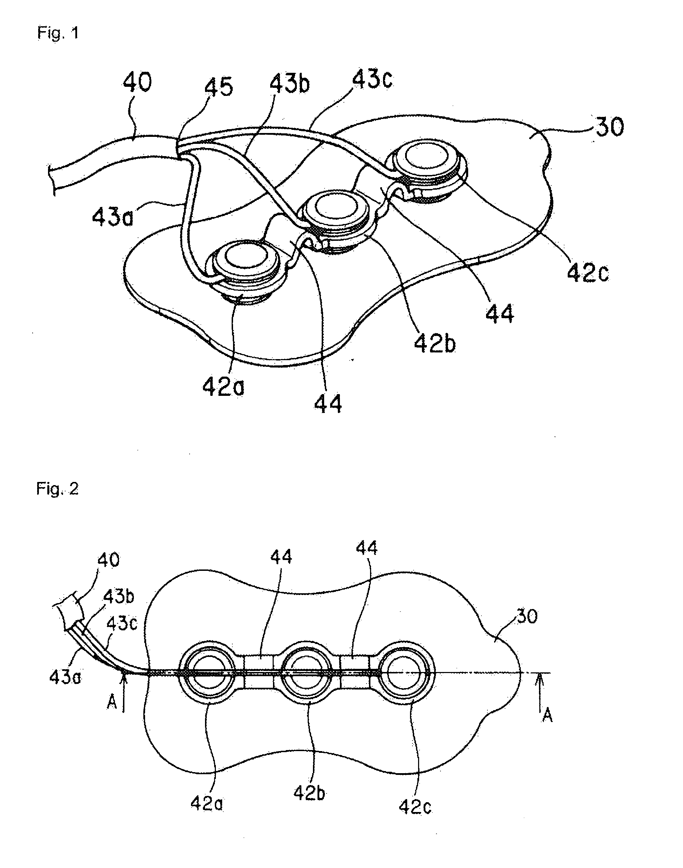

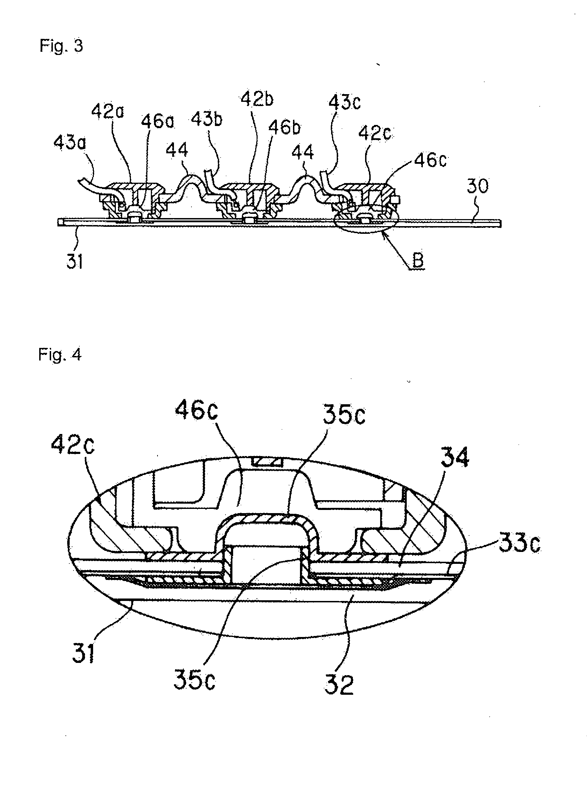

Fig. 1 is a perspective view schematically showing the pad 30 and the cord 40 in the first embodiment of the present invention. Fig. 2 is a plan view schematically showing the pad 30 and the cord 40 in the first embodiment of the present invention. Fig. 3 is a view schematically showing a cross-section taken along line A-A in Fig. 2. Fig. 4 is an enlarged view of portion B shown in Fig. 3.

The cord 40 will be described below.

The cord 40 according to the present embodiment is configured by three lead wires 43a, 43b, and 43c, where the end on the pad 30 side is arranged with three connection terminal portions 42a, 42b, and 42c, which are the connection terminal portion, of the same number respectively connected to the three lead wires 43a, 43b, and 43c to electrically connect to the pad 30.

- Each of the three connection terminal portions 42a, 42b, and 42c are independently arranged (separate from other connection terminal portions), and are arranged in a line (in one line, in series, in a straight line) as a connection terminal portion group by coupling the connection terminal portions 42a, 42b, and the connection terminal portions 42b, 42c through a coupling portion 44. In the figure, the three connection terminal portions 42a, 42b, and 42c are arranged in a substantially straight line, but are not limited thereto, and may be arranged in a curved form

The coupling portion 44 is configured by an elastomer as an elastic material. As shown in the figure, a band shaped (elongate shape, substantially flat plate-shape) member is formed to a curved shape. In the present embodiment, the connection terminal portions 42a, 42b, and the connection terminal portions 42b, 42c are coupled by the coupling portion 44 configured by one member, but is not limited thereto, and the connection terminal portions 42a, 42b, and the connection terminal portions 42b, 42c may be independently coupled by a coupling portion (or two coupling portions) configured by a plurality of members.

The three lead wires 43a, 43b, and 43c are united by a uniting means and collected as one up to the vicinity of the connection terminal portions 42a, 42b, and 42c from the plug 41, and branched by a branching portion 45 arranged at the vicinity of the connection terminal portions 42a, 42b, and 42c so as to be respectively connected to the connection terminal portions 42a, 42b, and 42c one by one.

The uniting means is not particularly limited as long as a plurality of lead wires can be collected to one. For instance, the plurality of lead wires may be collected to one cord by molding, or the plurality of lead wires may be united by wrapping a member capable of uniting such as adhesive tape and resin material.

The three connection terminal portions 42a, 42b, and 42c are respectively arranged with a depression-type snap 46a, 46b, and 46c for mechanically and electrically connecting to the pad 30.

The pad 30 according to the first embodiment of the present invention will now be described.

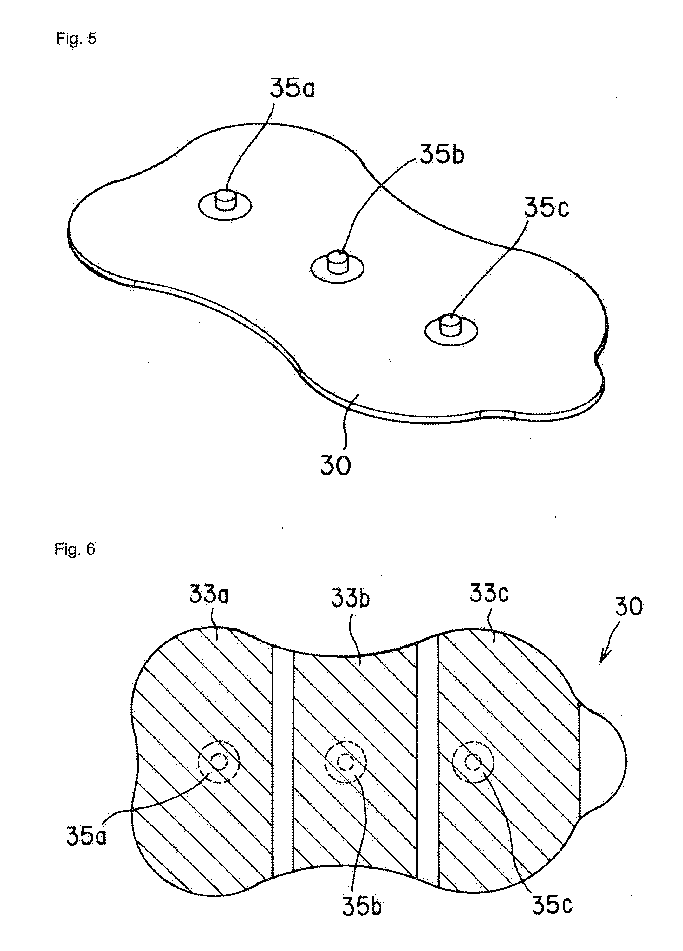

Fig. 5 is a perspective view schematically showing the pad 30 in the first embodiment of the present invention.

As shown in Fig. 4, the pad 30 according to the present embodiment has a gel 32 serving as a gel layer with an adhesive surface 31 to be attached to the treatment site at the surface of the living body, a conductive carbon layer 33c serving as a conductive electrode layer, and a base material sheet 34 formed with the conductive carbon layer 33c arranged in layers in order from the adhesive surface 31 side towards the connection terminal portion 42c side.

A projection-type snap 35c for connecting to the depression-type snap 46c of the connection terminal portion 42c is arranged at the portion connecting to the connection terminal portion 42c. The snap 35c is attached (caulked) from both sides of the base material sheet 34 formed with the conductive carbon layer 33c, where the gel 32 is arranged after attaching the snap 35c to the base material sheet 34.

The above description using Fig. 4 describes the portion corresponding to the connection terminal portion 42c of the cord 40 in the pad 30, but the configuration is the same other than for the conductive carbon layer and the snap, that is, the same for the gel 32 and the base material sheet 34 even for the portions corresponding to the connection terminal portions 42a and 42b in the pad 30. With regards to the conductive carbon layer and the snap, a conductive carbon layer 33a and a snap 35a, and a conductive carbon layer 33b and a snap 35b are arranged in correspondence to the connection terminal portions 42a, 42b, respectively.

Fig. 6 schematically shows a view, seen from the adhesive surface 31 side, of the pad 30 in the first embodiment of the present invention. In Fig. 6, hatching (shaded portion) is applied to a region corresponding to the conductive carbon layers 33a, 33b, and 33c for the sake of convenience of explanation.

In the pad 30 according to the present embodiment, the conductive carbon layer formed on one base material sheet 34 is arranged by being divided into three conductive carbon layers 33a, 33b, and 33c so as to respectively correspond to the three connection terminal portions 42a, 42b, and 42c arranged at the cord 40.

An integrated (one) gel 32 having a shape substantially following the outer shape of the base material sheet 34 is arranged on the three conductive carbon layers 33a, 33b, and 33c.

As described above, according to the present embodiment, the plurality of connection terminal portions 42a, 42b, and 42c, each of which are independently arranged, configure one connection terminal portion group by being coupled and connected by the coupling portion 44, thereby enhancing the flexibility between the connection terminal portions 42a, 42b, and 42c. In the pad 30 connected with the cord 40 as well, the flexibility can be maintained without the movement being regulated by the conduction cord as in the prior art.

Therefore, even if the pad 30 is attached to the treatment site of large curvature such as wrist, the ends of the pad 30 will not lift up, and the pad 30 can be more reliably and intimately attached to the treatment site.

Since the gel 32 arranged on the three conductive carbon layers 33a, 33b, and 33c is integrated, greater adhesive region with respect to the treatment site can be ensured, and the adhesive force of the pad 30 with respect to the treatment site can be further increased.

The gel does not need to be arranged for every conductive carbon layer by providing the integrated gel 32, and the generation of the gel can be simplified. Since the gel does not need to be arranged for every conductive carbon layer, the shape of the conductive carbon layer can be appropriately complexified.

The adhesive portion with respect to the treatment site is spread over the entire base material sheet by arranging the integrated gel 32, and thus the rigidity of the body of the pad 30 becomes substantially even. Thus, a portion that easily folds is eliminated even if the body of the pad 30 is bent. Therefore, the possibility of the pad 30 folding and the adhesive surfaces sticking to each other is further lowered when attaching the pad 30 to the treatment site.

The conductive carbon layers 33a, 33b, and 33c shown in Fig. 6 are arranged in a line and substantially in parallel so as to respectively correspond to the connection terminal portions 42a, 42b, and 42c, but the arrangement position of the conductive carbon layers 33a, 33b, and 33c is not limited thereto. Variants on the arrangement position of the conductive carbon layers 33a, 33b, and 33c will be described below.

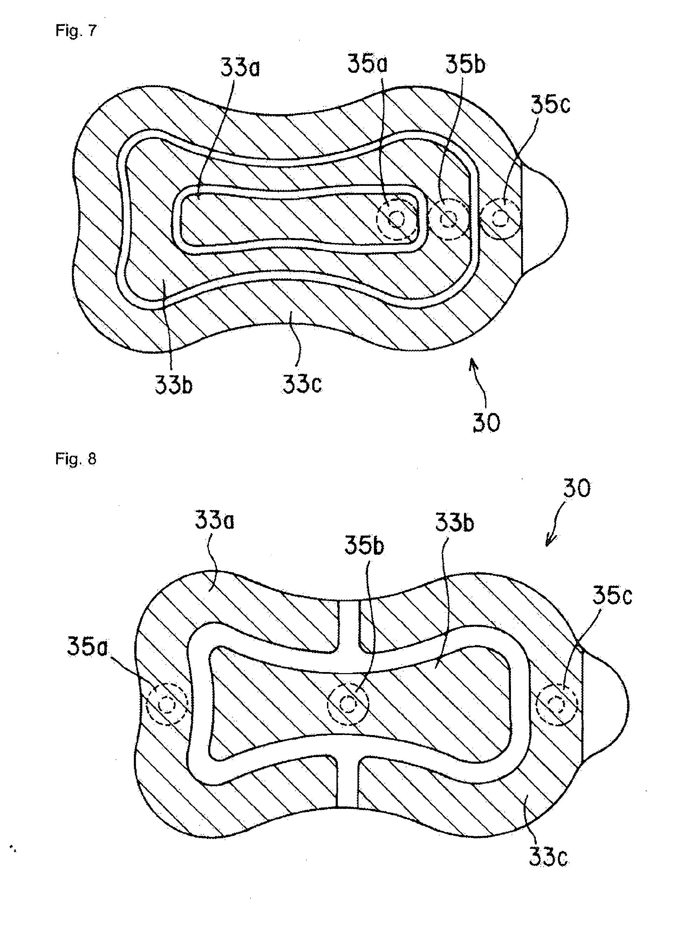

Figs. 7 to 9 are views describing variants on the arrangement of the conductive carbon layers 33a, 33b, and 33c.

In Fig. 7, an example where the three conductive carbon layers 33a, 33b, and 33c are substantially concentrically arranged is shown. That is, in Fig. 7, there is shown an example where the three conductive carbon layers 33a, 33b, 33c are arranged divided into three regions of a center region of the base material sheet 34, a first outer periphery region at the outer periphery of the center region, and a second outer periphery region at the outer periphery of the first outer periphery region.

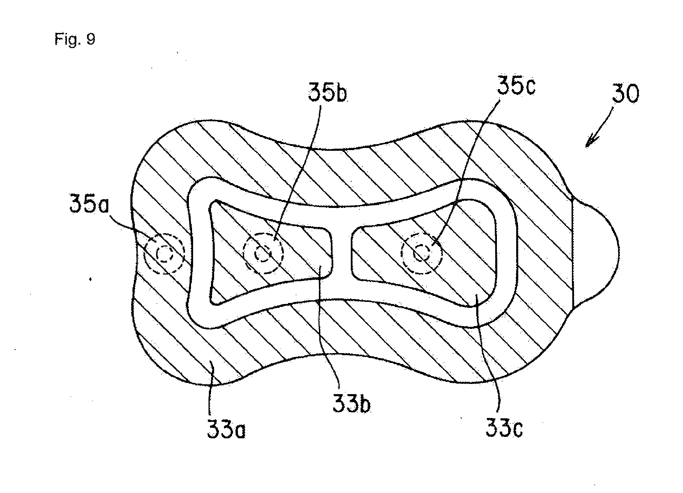

In the examples shown in Figs. 8 and 9, examples of dividing into two regions of the center region and an outer periphery region of the base material sheet 34 are shown, where the outer periphery region is further divided into two regions in the example shown in Fig. 8, and the center region is further divided into two regions in the example shown in Fig. 9.

That is, In Fig. 8, there is shown an example where the three conductive carbon layers 33a, 33b, and 33c are arranged divided into three regions of an outer periphery region on one end side, a center region, and an outer periphery region on the other end side of the base material sheet 34.

In Fig. 9, there is shown an example where the three conductive carbon layers 33a, 33b, and 33c are arranged divided into three regions of an outer periphery region, a center region on one end side, and a center region on the other end side of the base material sheet 34.

The arrangements of the conductive carbon layers shown in Figs. 7 to 9 are examples in the case where three conductive carbon layers 33a, 33b, and 33c are arranged, but the arrangement of the conductive carbon layers is not limited thereto. The arrangement of the conductive carbon layers may be appropriately set according to the specification of the low frequency treatment device.

Since the region to flow current to the electrode can be appropriately changed by such arrangement, the treatment site of the living body attached with the pad 30 can be changed, and a more effective treatment can be carried out.

At the coupling portion as well, the coupling portion 44 described in the first embodiment above is merely an example, and is not limited to the present embodiment.

Variants of the coupling portion will be shown below as second and eighth embodiments. The coupling portion of such embodiments is preferably made from an elastomer as the elastic material unless otherwise specifically stated. At the uniting means, a state in which three lead wires 43a, 43b, and 43c are united to the connection terminal portion group (state in which one cord uniting the three lead wires is connected to the coupling portion) without arranging the branching portion 45 as in the first embodiment is shown. The basic configuration is similar to the low frequency treatment device 10 of the first embodiment above, and thus the description thereof will not be given. With regards to the pad 30 and the connection terminal portions 42a, 42b, and 42c, the function and the connecting portion thereof are similar, and thus same reference numerals are used.

### (Second Embodiment)

A second embodiment of the present invention will be described below.

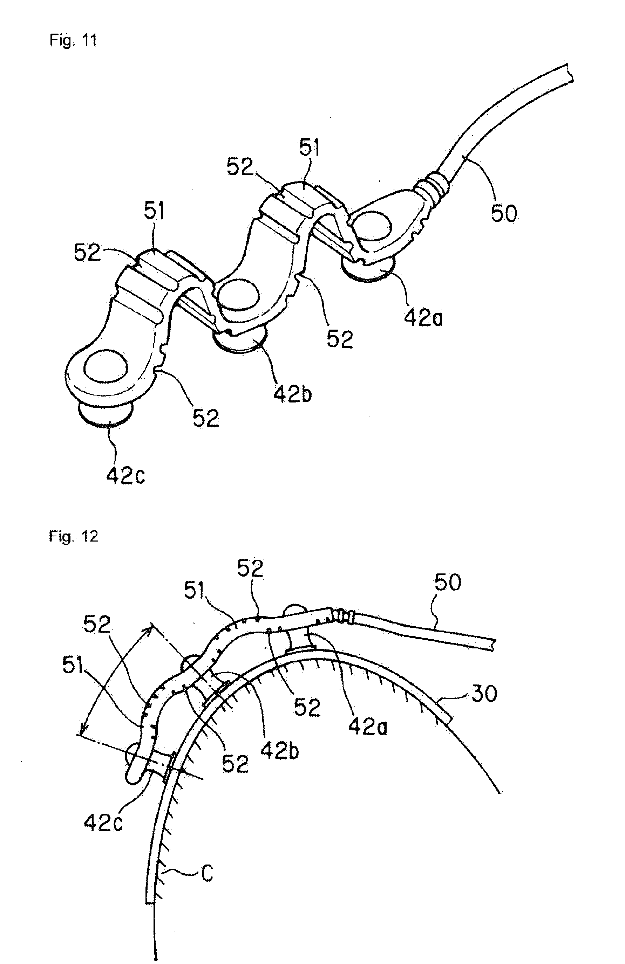

Fig. 11 is a perspective view schematically showing a cord 50 in the second embodiment of the present invention. Fig. 12 is a side view schematically showing the pad 30 and the cord 50 in the second embodiment of the present invention, showing a state in which the pad 30 attached with the cord 50 is attached to the treatment site of large curvature (indicated by shaded portion C).

A coupling portion 51 of the present embodiment is arranged in a band shape. In the present embodiment, a groove 52 is formed in the coupling portion 51 as a means for further enhancing the flexibility and the stretchability of the coupling portion.

The shape of the groove is not particularly limited, but in the present embodiment, the groove 52 extends in a direction substantially orthogonal to a virtual line connecting the connection terminal portions, as shown in Fig. 12. The groove 52 is preferably arranged at a portion of the coupling portion 51 where the extent of bend (deformation) is large, and is arranged on both surface sides of the band-shaped coupling portion 51, that is, on the side opposite to the side attached with the pad 30 at substantially the middle between the connection terminal portions, and on the side attached with the pad 30 near the connection terminal portion, in the present embodiment.

### (Third Embodiment)

A third embodiment of the present invention will be described below.

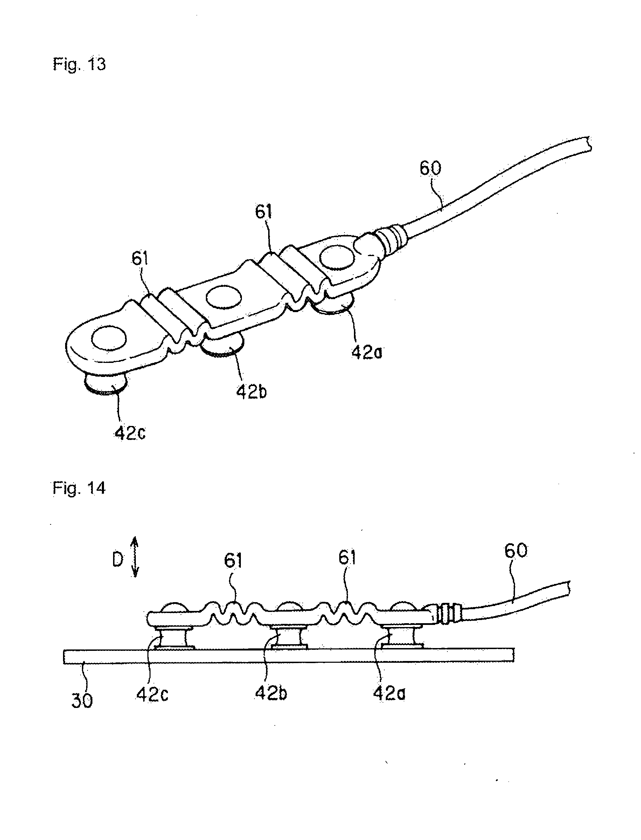

Fig. 13 is a perspective view schematically showing a cord 60 in the third embodiment of the present invention. Fig. 14 is a side view schematically showing the pad 30 and the cord 60 in the third embodiment of the present invention.

A coupling portion 61 of the present embodiment is also arranged in a band shape, similar to the coupling portion 51 of the second embodiment, and adopts a means for further enhancing the flexibility and the stretchability of the coupling portion. In the present embodiment, the coupling portion 61 has an accordion form as shown in the figure as a means for further enhancing the flexibility and the stretchability between the coupling portions. The accordion-shaped portion merely needs to be arranged at least at one part between the connection terminal portions.

### (Fourth Embodiment)

A fourth embodiment of the present invention will be described below.

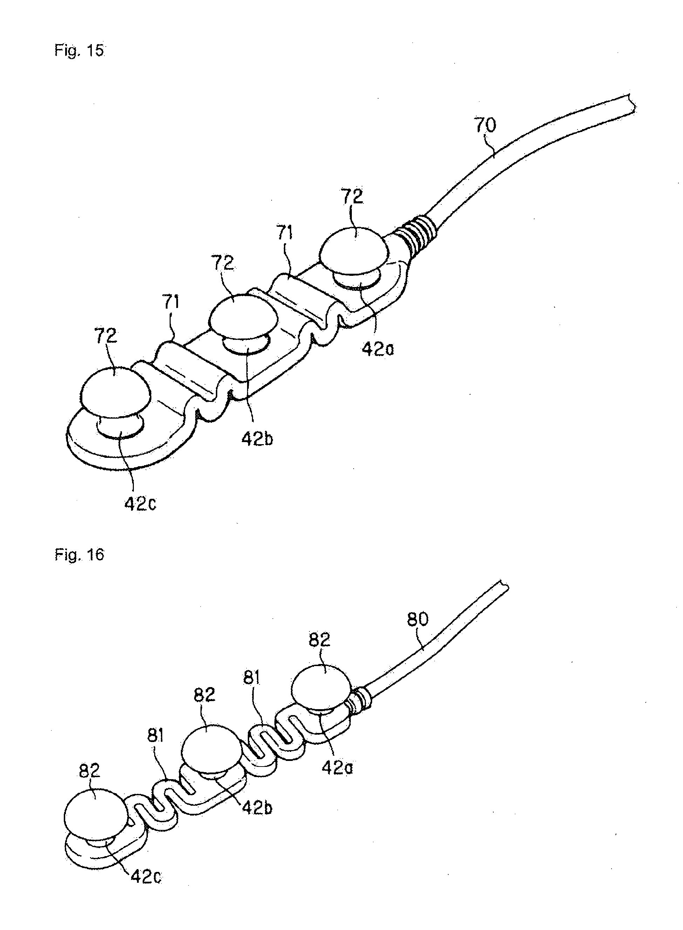

Fig. 15 is a perspective view schematically showing a cord 70 in the fourth embodiment of the present invention.

In a coupling portion 71 of the fourth embodiment of the present invention, an accordion-shaped portion is arranged similar to the third embodiment. In the present embodiment, projections 72 are arranged on the side opposite to the side attached with the pad 30 at the connection terminal portions 42a, 42b, and 42c, respectively.

In the present embodiment, by arranging the projection 72, the user can hold the projection 72 between the fingers when handling the cord 70, whereby the convenience and the handleability in handling the cord 70 are further enhanced. Since the projection 72 is arranged at the connecting portion of the cord 70 and the pad 30, the attachment workability in attaching the cord 70 to the pad 30 can be further enhanced. Furthermore, the attachment work in attaching the pad 30 to the treatment site is more easily carried out.

### (Fifth Embodiment)

A fifth embodiment of the present invention will be described below.

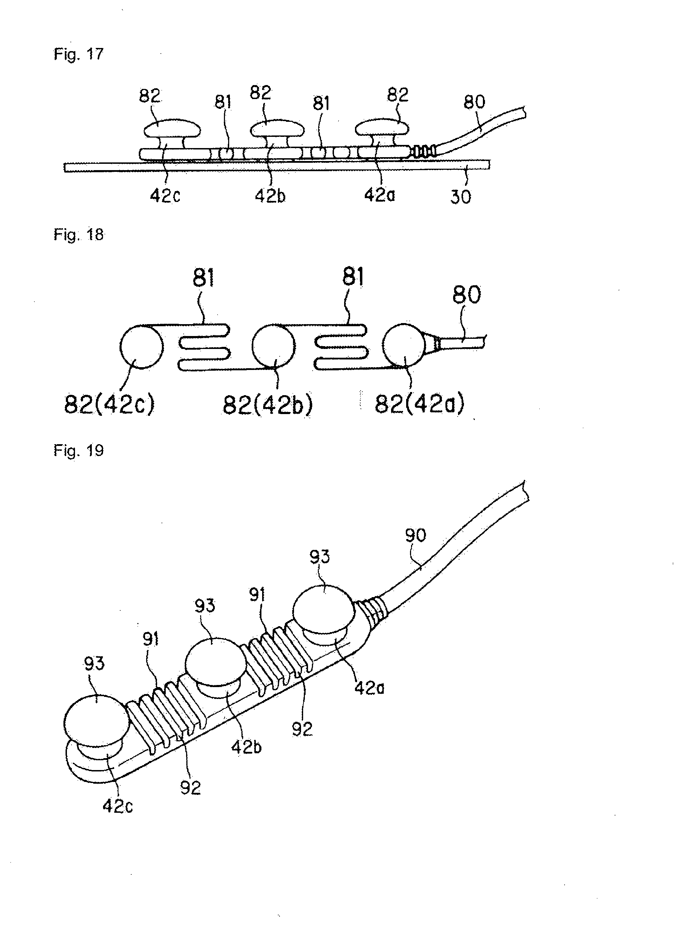

Fig. 16 is a perspective view schematically showing a cord 80 in the fifth embodiment of the present invention. Fig. 17 is a side view schematically showing the pad 30 and the cord 80 in the fifth embodiment of the present invention.

At a coupling portion 81 of the present embodiment, the accordion-shape is adopted as a means for further enhancing the flexibility and the stretchability of the coupling portion, similar to the coupling portions 61, 71 of the third and fourth embodiments, but a rod-shaped member (member which vertical and horizontal lengths are barely changed in cross-section) of accordion shape is used in the present embodiment as opposed to the coupling portions 61, 71 of the third and fourth embodiments having a band-shape. The accordion-shaped portion merely needs to be arranged on at least one part between the connection terminal portions.

In the coupling portions 61, 71 of the third and fourth embodiments, the accordion-shape is formed by repeatedly arranging irregularities in a direction substantially orthogonal to the sheet surface of the substrate sheet 34 of the pad 30 attached with the cord (direction of arrow D shown in Fig. 14, between the side attached to the pad 30 and the opposite side), but in the present embodiment, the irregularities may be repeatedly arranged within the sheet surface of the substrate sheet 34.

Furthermore, in the present embodiment, projections 82 are arranged on the side opposite to the side attached with the pad 30 at the connection terminal portions 42a, 42b, and 42c, respectively. Each of the projections 82 is similar to the projection 72 of the fourth embodiment.

Fig. 18 is a view schematically showing a variant with respect to an accordion-shape of the present embodiment.

The accordion-shape of the coupling portion 81 formed so as to repeat irregularities within the sheet surface of the substrate sheet 34 may be a shape as shown in Fig. 18.

### (Sixth Embodiment)

A sixth embodiment of the present invention will be described below.

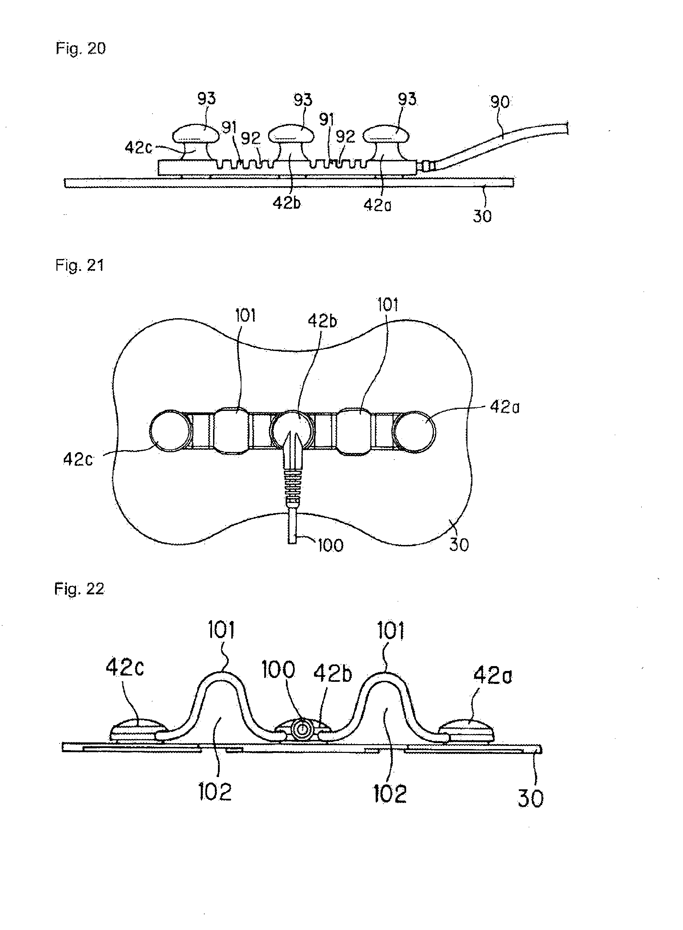

Fig. 19 is a perspective view schematically showing a cord 90 in the sixth embodiment of the present invention. Fig. 20 is a side view schematically showing the pad 30 and the cord 90 in the sixth embodiment of the present invention.

In the present embodiment, projections 93 are arranged on the side opposite to the side attached with the pad 30 at the connection terminal portion 42a, 42b, and 42c, respectively. Each of the projections 93 is similar to the projection 72 of the fourth embodiment.

In the sixth embodiment of the present invention, a coupling portion 91 has a shape in which a groove 92 is formed on a flat plate-shape. In the present embodiment, it is not in a curved form in a natural state, and the groove is formed only on the side opposite to the side attached with the pad 30 of the coupling portion 91.

The shape of the groove 92 is not particularly limited, but is a groove extending in a direction substantially orthogonal to the virtual line connecting the connection terminal portions, similar to the second embodiment.

The cord 90 that can flexibly deform along the pad 30 is thereby configured by arranging the coupling portion 91.

### (Seventh Embodiment)

A seventh embodiment of the present invention will be described below.

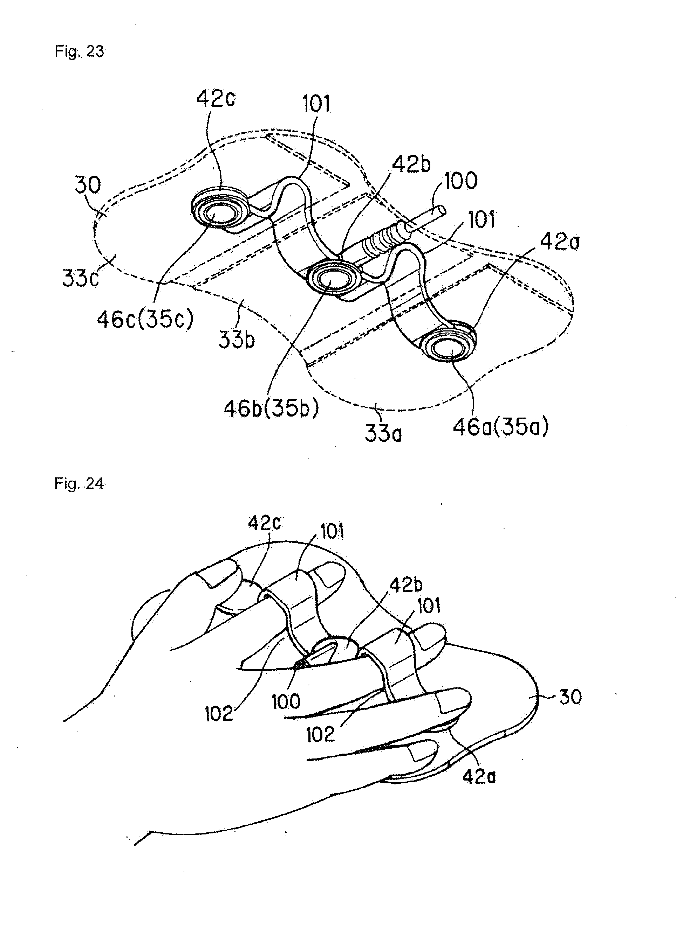

Fig. 21 is a plan view schematically showing a cord 100 in the seventh embodiment of the present invention. Fig. 22 is a side view schematically showing the pad 30 and the cord 100 in the seventh embodiment of the present invention. Fig. 23 schematically shows a view, seen from the connecting portion side with the pad 30, of the cord 100 in the seventh embodiment of the present invention, where the pad 30 attached to the cord 100 and the three conductive carbon layers 33a, 33b, and 33c arranged on the pad 30 are shown with broken lines.

In the seventh embodiment of the present invention, the position at where the lead wire is connected to the connection terminal portion group, and the connecting direction of the lead wire with respect to the connection terminal portion group are different with respect to the second to sixth embodiments described above.

In the embodiments described above, connection is made to the connection terminal portion at the end of the connection terminal portion group arranged in a line, and the connecting direction of the lead wire at the connecting portion is substantially the same direction as the aligned direction of the connection terminal portion group (direction connecting the connection terminal portions).

In the present embodiment, on the other hand, connection is made to the connection terminal portion positioned at the middle of the connection terminal portion group arranged in a line, and the lead wire is arranged so as to cross such a connection terminal portion, that is, so that the connecting direction of the lead wire at the connecting portion is in a direction substantially orthogonal to the aligned direction of the connection terminal portion group.

The two-method cord in which the connecting direction of the lead wire with respect to the connection terminal portion group is different is appropriately selected and applied according to the specification of the low frequency treatment device. The cord of both methods may be applied to one low frequency treatment device, and appropriately selected according to the treatment site.

A coupling portion 101 of the present embodiment has a band shape and is arranged in a curved shape, where a gap 102 forms between the coupling portion 101 and the pad 30 when the cord 100 is attached to the pad 30.

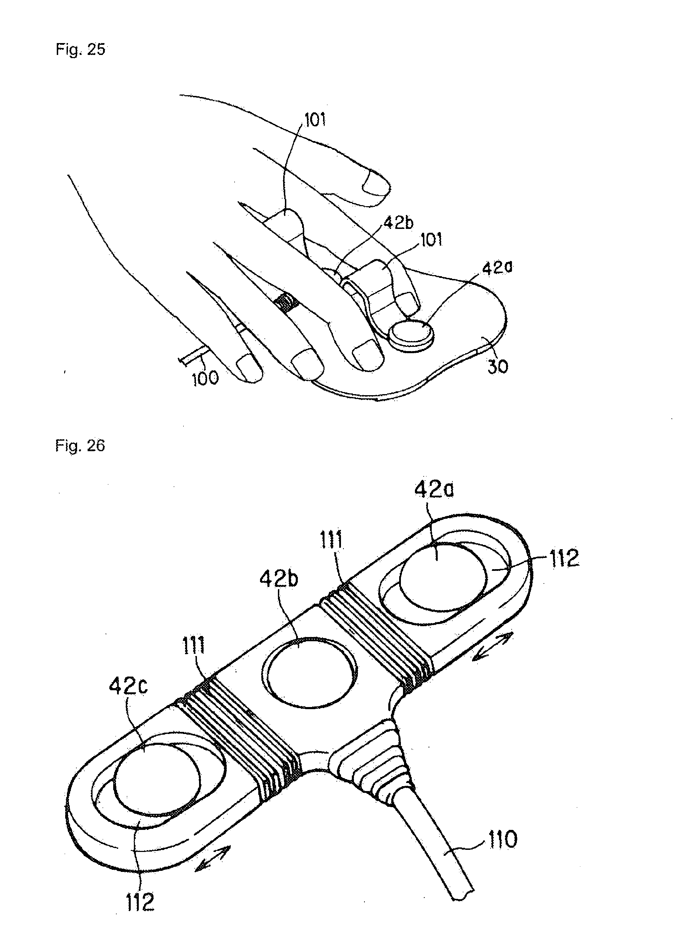

Figs. 24 and 25 are views schematically showing a state in which the user holds the pad 30 by hand.

As shown in Fig. 24, the user can hold the pad 30 by inserting the fingers to the gap 102 between the coupling portion 101 and the pad 30. The connection terminal portion group arranged in a line can be easily sandwiched between the fingers, as shown in Fig. 25, by forming the gap 102 to a size the finger of the user can be inserted. The gap 102 may not be formed as large to the size the finger of the user can be inserted, and may be formed to a size the coupling portion 101 can be sandwiched between the fingers.

According to the present embodiment, when the user holds the pad 30 by hand, the pad 30 can be attached to the treatment site with the finger inserted to the coupling portion or the coupling portion sandwiched between the fingers without carrying out the movement of grabbing the pad 30 by hand. Therefore, the convenience in handling the pad 30 can be enhanced, and the attachment workability in attaching the pad 30 to the treatment site can be enhanced.

Furthermore, since the finger (fingertip) of the user is positioned at the coupling portion arranged at the position corresponding to between the conductive carbon layers 33a, 33b, and 33c, the pad 30 can be more reliably attached to the desired treatment site.

### (Eighth embodiment)

An eighth embodiment of the present invention will be described below.

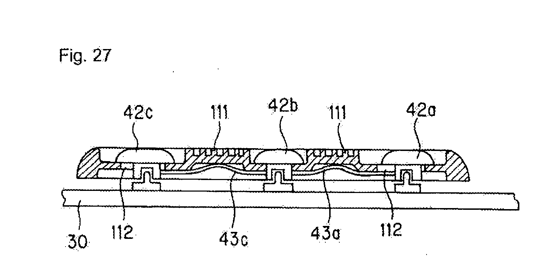

Fig. 26 is a plan view schematically showing a cord 110 in the eighth embodiment of the present invention. Fig. 27 is a side view schematically showing the pad 30 and the cord 110 in the eighth embodiment of the present invention.

In the eighth embodiment of the present invention, the connection terminal portions 42a, 42c are held using a horizontally long gap 112 so as to be movable to a coupling portion 111.

In the present embodiment, the connection terminal portions 42a, 42c are arranged movable in the aligned direction (direction of arrow shown in Fig. 26) of the connection terminal portion group. A groove similar to the groove 92 of the sixth embodiment described using Figs. 19 and 20 is formed in the coupling portion 111.

The lead wires 43a, 43c connected to the movably arranged connection terminal portions 42a, 42c are movably arranged so as not to inhibit the movement of the connection terminal portions 42a, 42c. Here, the lead wires 43a, 43c are preferably arranged inside the coupling portion 111, but may be arranged towards the side attached with the pad 30 than the coupling portion 111. That is, the lead wires 43a, 43c merely need to be arranged so as not to lower the workability of the attachment task of the user when the pad 30 is attached to the cord 110.

According to the present embodiment, even if the pad 30 is attached to the treatment site of large curvature, the connection terminal portions 42a, 42c can move with the bending movement of the pad 30 in addition to the deformation of the coupling portion 111 as the connection terminal portions 42a, 42c are movably held at the coupling portion 111. Therefore, the movement of the cord 110 can be more flexibly responded with respect to the movement of the pad 30.

Therefore, in the present embodiment, not only due to the flexibility of the coupling portion 111, but also due to the movement of the connection terminal portions 42a, 42c with respect to the coupling portion 111, the ends of the pad 30 does not lift up and the pad 30 can be more reliably and intimately attached to the treatment site even if the pad 30 is attached to the treatment site of large curvature such as a wrist. The application range of material of an elastic material constituting the coupling portion 111 then can be extended. That is, the coupling portion 111 may be made of resin material and the like in the present embodiment.

## Claims

1. A low frequency treatment device conduction cord comprising:
a plurality of connection terminal portions arranged to contact to and separate from a plurality of electrodes arranged on a pad;
a plurality of lead wires, connected to each of the plurality of connection terminal portions, for electrically connecting a low frequency treatment device body and the pad; and
a coupling portion, formed to a band-shape from an elastic material, for coupling the connection terminal portions; wherein
the plurality of connection terminal portions is independently arranged, and coupled by the band-shaped coupling portion to form one connection terminal portion group.

2. The conduction cord for low frequency treatment device according to claim 1, wherein the coupling portion has a curved shape between the connection terminal portions.

3. The conduction cord for low frequency treatment device according to claim 2, wherein the coupling portion is arranged in a curved state to enable a user to insert a finger between the coupling portion and the pad and hold the pad with the plurality of connection terminal portions connected to the plurality of electrodes arranged on the pad.

4. The conduction cord for low frequency treatment device according to claim 2, wherein the coupling portion is arranged in a curved state to enable a user to hold the pad between the fingers with the plurality of connection terminal portions connected to the plurality of electrodes arranged on the pad.

5. The conduction cord for low frequency treatment device according to any one of claims 1 to 4, further comprising:
a uniting means for uniting the plurality of lead wires from the low frequency treatment device body side to the vicinity of the connection terminal portion group; wherein
the plurality of lead wires are branched one by one at the vicinity of the connection terminal portion group, and connected to each of the plurality of connection terminal portions.

6. The conduction cord for low frequency treatment device according to any one of claims 1 to 4, further comprising:
a uniting means for uniting the plurality of lead wires from the low frequency treatment device body side to the connection terminal portion group; wherein
the plurality of lead wires united to the connection terminal portion group by the uniting means are arranged so as to pass through an interior of the coupling portion and connect to the corresponding connection terminal portion.

7. The conduction cord for low frequency treatment device according to any one of claims 1 to 6, wherein a projection projecting to be held by the finger is arranged on a side opposite to the side connecting to the plurality of electrodes arranged on the pad at the plurality of connection terminal portions.

8. The conduction cord for low frequency treatment device according to any one of claims 1 to 7, wherein the plurality of connection terminal portions configure one connection terminal portion group formed in a line.

9. The conduction cord for low frequency treatment device according to clam 8, wherein a direction in which the plurality of lead wires united by the uniting means is directed towards the connection terminal portion group is orthogonal to an aligned direction of the connection terminal portion group formed in a line.

10. The conduction cord for low frequency treatment device according to any one of claims 1 to 9, wherein at least one connection terminal portion of the plurality of connection terminal portions is arranged to be movable with respect to the coupling portion.

11. A low frequency treatment device comprising an electrode pad attached with the conduction cord according to any one of claims 1 to 10, the electrode pad comprising:
a plurality of conductive electrode layers, arranged on a base material sheet, respectively configuring an electrode connected to the plurality of connection terminal portions; and
a gel layer having an adhesive surface integrally formed by gel-like material, arranged so as to cover the plurality of conductive electrode layers on the base material sheet, and provided for attachment to a treatment site.

## Patentansprüche

1. Leitungskabel für Niederfrequenzbehandlungsgerät, umfassend:
eine Vielzahl an Verbindungsanschlussabschnitten, welche angeordnet sind, um mit einer Vielzahl an Elektroden, die auf einem Pad angeordnet sind, in Kontakt zu treten und von diesen getrennt zu werden,
eine Vielzahl an Zuleitungsdrähten, welche mit jedem der Vielzahl an Verbindungsanschlussabschnitten verbunden sind, um ein Gehäuse des Niederfrequenzbehandlungsgeräts und das Pad elektrisch zu verbinden, und
einen Kopplungsabschnitt, der in Form eines Bandes aus einem elastischen Material gebildet ist, um die Verbindungsanschlussabschnitte zu verkoppeln, wobei
die Vielzahl an Verbindungsanschlussabschnitten unabhängig angeordnet und durch den bandförmigen Kopplungsabschnitt verkoppelt ist, um eine Verbindungsanschlussabschnittsgruppe zu bilden.

2. Leitungskabel für Niederfrequenzbehandlungsgerät gemäß Anspruch 1, wobei der Kopplungsabschnitt zwischen den Verbindungsanschlussabschnitten eine Kurvenform aufweist.

3. Leitungskabel für Niederfrequenzbehandlungsgerät gemäß Anspruch 2, wobei der Kopplungsabschnitt kurvenförmig angeordnet ist, um es einem Nutzer zu ermöglichen, einen Finger zwischen den Kopplungsabschnitt und das Pad einzuführen und das Pad mit der Vielzahl an Verbindungsanschlussabschnitten in Verbindung mit der Vielzahl an auf dem Pad angeordneten Elektroden zu halten.

4. Leitungskabel für Niederfrequenzbehandlungsgerät gemäß Anspruch 2, wobei der Kopplungsabschnitt kurvenförmig angeordnet ist, um es einem Nutzer zu ermöglichen, das Pad mit der Vielzahl an Verbindungsanschlussabschnitten in Verbindung mit der Vielzahl an auf dem Pad angeordneten Elektroden zwischen den Fingern zu halten.

5. Leitungskabel für Niederfrequenzbehandlungsgerät gemäß irgendeinem der Ansprüche 1 bis 4, ferner umfassend:
ein Vereinigungsmittel zum Vereinigen der Vielzahl an Zuleitungsdrähten von der Seite des Gehäuses des Niederfrequenzbehandlungsgeräts bis in die Nähe der Verbindungsanschlussabschnlttsgruppe, wobei
die Vielzahl an Zuleltungsdrähten In der Nähe der Verbindungsanschlussabschnittsgruppe voneinander abzweigen und mit jedem der Vielzahl an Verbindungsanschlussabschnitten verbunden sind.

6. Leitungskabel für Niederfrequenzbehandlungsgerät gemäß irgendeinem der Ansprüche 1 bis 4, ferner umfassend:
ein Vereinigungsmittel zum Vereinigen der Vielzahl an Zuleitungsdrähten von der Seite des Gehäuses des Niederfrequenzbehandlungsgeräts bis zu der Verbindungsanschlussabschnittsgruppe, wobei
die Vielzahl an Zuleitungsdrähten, die durch das Vereinigungsmittel bis zu der Verbindungsanschlussabschnittsgruppe vereinigt werden, derart angeordnet sind, dass sie durch das Innere des Kopplungsabschnitts geführt werden und mit dem entsprechenden Verbindungsanschlussabschnitt verbunden sind.

7. Leitungskabel für Niederfrequenzbehandlungsgerät gemäß irgendeinem der Ansprüche 1 bis 6, wobei ein Vorsprung, welcher derart vorsteht, dass er mit dem Finger gehalten werden kann, auf einer Seite gegenüberliegend der Seite, auf der die Vielzahl der auf dem Pad angeordneten Elektroden mit der Vielzahl an Verbindungsanschlussabschnitten verbunden sind, angeordnet ist.

8. Leitungskabel für Niederfrequenzbehandlungsgerät gemäß irgendeinem der Ansprüche 1 bis 7, wobei die Vielzahl an Verbindungsanschlussabschnitten eine Verbindungsanschlussabschnittsgruppe bilden, die in einer Reihe ausgebildet ist.

9. Leitungskabel für Niederfrequenzbehandlungsgerät gemäß Anspruch 8, wobei eine Richtung, in der die Vielzahl an durch das Vereinigungsmittel vereinigten Zuleitungsdrähten in Richtung Verbindungsanschlussabschnittsgruppe geführt werden, orthogonal zu der Richtung, in der die in einer Reihe ausgebildete Verbindungsanschlussabschnittsgruppe angeordnet ist, vorliegt.

10. Leitungskabel für Niederfrequenzbehandlungsgerät gemäß irgendeinem der Ansprüche 1 bis 9, wobei wenigstens ein Verbindungsanschlussabschnitt aus der Vielzahl an Verbindungsanschlussabschnitten derart angeordnet ist, dass er in Bezug auf den Kopplungsabschnitt beweglich ist.

11. Niederfrequenzbehandlungsgerät umfassend ein Elektrodenpad, welches mit dem Leitungskabel gemäß irgendeinem der Ansprüche 1 bis 10 verbunden ist, wobei das Elektrodenpad umfasst:
eine Mehrzahl an leitfähigen Elektrodenschichten, die auf einer Basismaterialschicht angeordnet sind und jeweils eine Elektrode bilden, die mit der Vielzahl an Verbindungsanschlussabschnitten verbunden Ist, und
eine Gelschicht, welche eine adhäsive Oberfläche aufweist, die integral durch Gel-artiges Material gebildet wird, und derart angeordnet ist, dass sie die Vielzahl an leitfähigen Elektrodenschichten auf der Basismaterialschicht bedeckt, und welche vorgesehen ist, um an einer Behandlungsstelle anzuhaften.

## Revendications

1. Cordon conducteur de dispositif de traitement à basse fréquence, comprenant :
une pluralité de parties bornes de connexion agencées pour entrer en contact avec une pluralité d'électrodes agencées sur une plage et se séparer de celles-ci ;
une pluralité de fils conducteurs, connectés à chacune parmi la pluralité de parties bornes de connexion, pour connecter électriquement un corps de dispositif de traitement à basse fréquence et la plage ; et
une partie de couplage, formée en une bande à partir d'un matériau élastique, pour coupler les parties bornes de connexion ; dans lequel
la pluralité de parties bornes de connexion sont agencées indépendamment, et couplées par la partie de couplage en forme de bande pour former un groupe de parties bornes de connexion.

2. Cordon conducteur pour dispositif de traitement à basse fréquence selon la revendication 1, dans lequel la partie de couplage présente une forme incurvée entre les parties bornes de connexion.

3. Cordon conducteur pour dispositif de traitement à basse fréquence selon la revendication 2, dans lequel la partie de couplage est agencée dans un état incurvé pour permettre à un utilisateur d'insérer un doigt entre la partie de couplage et la plage et retenir la plage avec la pluralité de parties bornes de connexion connectées à la pluralité d'électrodes agencées sur la plage.

4. Cordon conducteur pour dispositif de traitement à basse fréquence selon la revendication 2, dans lequel la partie de couplage est agencée dans un état incurvé pour permettre à un utilisateur de tenir la plage entre les doigts avec la pluralité de parties bornes de connexion connectées à la pluralité d'électrodes agencées sur la plage.

5. Cordon conducteur pour dispositif de traitement à basse fréquence selon une quelconque des revendications 1 à 4, comprenant en outre :
un moyen d'union pour unir la pluralité de fils conducteurs du côté corps de dispositif de traitement à basse fréquence au voisinage du groupe de parties bornes de connexion ; dans lequel
la pluralité de fils conducteurs sont branchés un par un dans le voisinage du groupe de parties bornes de connexion, et connectés à chacune parmi la pluralité de parties bornes de connexion.

6. Cordon conducteur pour dispositif de traitement à basse fréquence selon une quelconque des revendications 1 à 4, comprenant en outre :
un moyen d'union pour unir la pluralité de fils conducteurs du côté corps de dispositif de traitement à basse fréquence au groupe de parties bornes de connexion ; dans lequel
la pluralité de fils conducteurs unis au groupe de parties bornes de connexion par le moyen d'union sont agencés afin de passer à travers un intérieur de la partie de couplage et se connecter à la partie borne de connexion correspondante.

7. Cordon conducteur pour dispositif de traitement à basse fréquence selon une quelconque des revendications 1 à 6, dans lequel une saillie faisant saillie pour être tenue par le doigt est agencée sur un côté opposé au côté se connectant à la pluralité d'électrodes agencées sur la plage au niveau de la pluralité de parties bornes de connexion.

8. Cordon conducteur pour dispositif de traitement à basse fréquence selon une quelconque des revendications 1 à 7, dans lequel la pluralité de parties bornes de connexion configurent un groupe de parties bornes de connexion formées en une ligne.

9. Cordon conducteur pour dispositif de traitement à basse fréquence selon la revendication 8, dans lequel une direction dans laquelle la pluralité de fils conducteurs unis par le moyen d'union sont dirigés vers le groupe de parties bornes de connexion est orthogonale à une direction alignée du groupe de parties bornes de connexion formées en une ligne.

10. Cordon conducteur pour dispositif de traitement à basse fréquence selon une quelconque des revendications 1 à 9, dans lequel au moins une partie borne de connexion parmi la pluralité de parties bornes de connexion est agencée pour être mobile par rapport à la partie de couplage.

11. Dispositif de traitement à basse fréquence comprenant une plage d'électrode fixée au cordon conducteur selon une quelconque des revendications 1 à 10, la plage d'électrode comprenant :
une pluralité de couches électrodes conductrices, agencées sur une feuille de matériau de base, configurant respectivement une électrode connectée à la pluralité de parties bornes de connexion ; et
une couche de gel comportant une surface adhésive formée de façon intégrée par un matériau similaire à du gel, agencée afin de couvrir la pluralité de couches électrodes conductrices sur la feuille de matériau de base, et prévue pour la fixation à un site de traitement.
